# EUROPEAN PATENT APPLICATION

(11) **EP 4 814 105 A1**
(43) Date of publication of application: **30.09.2026**
(21) Application number: 24896301.9
(22) Date of filing: 15.11.2024
(51) Int. Cl.: C12N 1/20, C12R 1/01

(54) **CULTURE MEDIUM AND METHOD FOR CULTURING AKKERMANSIA MUCINIPHILA**

(30) Priority: 30.11.2023 CN 202311622616
(71) Applicant: Beijing Quantihealth Technology Co., Ltd., Beijing 100070 (CN)
(72) Inventor: KANG, Guokai, Beijing 100070 (CN); XU, Wenyi, Beijing 100070 (CN); ZHANG, Yinghui, Beijing 100070 (CN); JIAO, Lin, Beijing 100070 (CN); LI, Xiaolin, Beijing 100070 (CN); ZHAO, Bowen, Beijing 100070 (CN); MA, Liang, Beijing 100070 (CN); LIU, Yanhong, Beijing 100070 (CN); LI, Hang, Beijing 100070 (CN)
(74) Representative: Bayramoglu et al.
(86) International application number: PCT/CN2024/132452
(87) International publication number: WO 2025/113210

(57) **Abstract**

Disclosed are a method for optimizing a bacterial culture medium based on a metabolic network model, a culture medium for *Akkermansia muciniphila* obtained thereby, and related applications thereof. The medium optimized by the method reduces redundant components present in the prior art media, has a simple composition and low cost, and can more directly and effectively promote high-density culture of bacteria. The resulting culture medium for *Akkermansia muciniphila* can increase the viable cell count to above 10¹⁰ cfu/mL, enabling industrial production of *Akkermansia muciniphila.*

## Description

### Technical Field

The present invention relates to the field of microbial culture. In particular, the present invention relates to a high-density culture medium for *Akkermansia muciniphila* (*A. muciniphila*), a method of preparing the culture medium, and relevant applications thereof.

### Background Technology

*Akkermansia muciniphila* is a Gram-negative bacterium isolated from the human intestinal tract. It is anaerobic yet tolerant to oxygen at the nanomolar level. Abundantly present in the human gut, it accounts for 3% to 5% of the total gut microbiota and colonizes the intestinal mucus layer. This bacterium can utilize mucin as the sole carbon and nitrogen source. As the only member of the phylum Verrucomicrobia inhabiting the intestinal tract of mammals, and because it can be easily detected via its 16S rRNA gene sequences, numerous studies have demonstrated that fluctuations in the abundance of the genus *Akkermansia* in the gut are associated with host health and diseases.

The gut microbiota is closely associated with various host physiological processes, such as energy intake, immune system maturation, and hormone secretion. As an important member of gut microbiota, *Akkermansia muciniphila* is regarded as one of the "next-generation probiotics." Changes in its abundance are significantly associated with multiple physiological disorders, including obesity, diabetes, and inflammatory bowel disease. Numerous studies have shown that administration of either viable *Akkermansia muciniphila* or pasteurized inactivated cells to mice can reduce body weight and fat accumulation, lower serum triglyceride and fasting blood glucose levels, and improve insulin sensitivity in mice. The mechanism by which *Akkermansia muciniphila* regulates glucose and lipid metabolism can be explained by its modulatory effects on barrier function and immune responses, with its metabolites serving as the material basis. Furthermore, potential cross-feeding and nutritional interactions between *Akkermansia muciniphila* and butyrate-producing bacteria may boost butyrate synthesis. These short-chain fatty acids can activate GPR43 or GPR41, thereby further regulating lipid and glucose metabolism.

Numerous studies have shown that colitis and inflammatory bowel disease (IBD) are associated with altered abundance of *Akkermansia muciniphila*. The abundance of this bacterium drops markedly in patients with IBD. In contrast, higher levels of *Akkermansia muciniphila* have been detected in patients with constipation-predominant irritable bowel syndrome (C-IBS) compared with healthy individuals. Animals colonized with C-IBS microbiota show improved recovery from dextran sulfate sodium (DSS)-induced colitis. Pre-treatment with *Akkermansia muciniphila* also alleviates the severity of colitis in conventional C57BL/6 mice and rats associated with human microbiota. These findings demonstrate the anti-inflammatory effects of gut microbiota in C-IBS patients, particularly *Akkermansia muciniphila*.

Meanwhile, studies have indicated that *Akkermansia muciniphila* may play a role in the development and regulation of autoimmune diseases. A gluten-free diet fed to NOD mice was associated with a lower incidence of spontaneous type I diabetes, along with an increased abundance of *Akkermansia muciniphila*. In cancer patients who responded to PD-1 therapy, the abundance of this bacterium was markedly higher than in those who showed no response to the treatment.

Despite the multiple health benefits of *Akkermansia muciniphila*, major bottlenecks in its large-scale culturing remain to be addressed. Derrien et al. reported a culturing method. Chinese Patents No. CN107849093B and CN107384828B further optimized the method established by Derrien et al. Nevertheless, all three culture media are developed on the basis of the basal medium proposed by Derrien's team. This basal medium contains an extremely complex composition consisting of approximately 30 chemical compounds. After fermentation, centrifugation becomes difficult in downstream processing, resulting in an extremely low centrifugation recovery rate and substantial biomass loss, which hinders industrial production. In addition, the medium is rich in Ca²⁺, and calcium salt precipitates form after high-temperature and high-pressure sterilization, causing severe adverse impacts on production equipment. Furthermore, the culturing methods disclosed by Derrien et al. and in Chinese Patent No. CN107384828B adopt animal-derived ingredients, which contain potential allergens and other undesirable components for food and pharmaceutical production. Researchers have also found that the growth of *Akkermansia muciniphila* is affected by the initial liquid level of the medium during conventional culturing. Chinese Patent No. CN112342151B discloses another culturing method that is not restricted by the liquid level. However, its improvement is based on the BHI medium, which has complex components, abundant animal-derived ingredients, and high costs, and no in-depth research on the high-density fermentation of *Akkermansia muciniphila* has been conducted. Another culture medium is disclosed in Chinese Patent No. CN201910265701.0. Though its composition is relatively simplified, it requires a culturing period of up to three days and yields a low bacterial count, with no exploration into high-density fermentation either. Plovier H et al. published a synthetic medium in their study (A purified membrane protein from Akkermansia muciniphila or the pasteurized bacterium improves metabolism obese and diabetic mice [J]. Nature Medicine, 2017, 23(1): 107-113). This synthetic medium achieves the same culturing efficiency as mucin-based media without altering the physiological properties of *Akkermansia muciniphila*. Even so, it includes as many as 33 compounds and fails to resolve the existing problems, including complex composition, massive precipitation after sterilization, low centrifugation recovery rate, and difficult downstream processing. Moreover, it contains Na₂S·9H₂O, a substance prohibited for use in food and pharmaceutical production.

Due to the particularity of isolating *Akkermansia muciniphila* - which is obtained using mucin-containing media - researchers have been trapped in a fixed mindset when developing culture media for this bacterium. To date, none of the published patents or patent applications have broken away from mucin derivatives, namely glucose, galactose, fucose, N-acetyl-D-glucosamine, and N-acetyl-D-galactosamine. Among these substances, glucose and N-acetyl-D-glucosamine are the most widely used. Nevertheless, during high-temperature and high-pressure sterilization, these components undergo intense Maillard reactions with proteins and amino acids in nitrogen sources. This leads to massive loss of nutrients and severe browning of the culture medium. In addition, the products generated from Maillard reactions exert certain bacteriostatic effects, which may hinder the growth of *Akkermansia muciniphila*.

Conventional strategies for optimizing culture media for *Akkermansia muciniphila* are largely empirical. They fail to accurately identify the nutrients essential for bacterial growth and thus consume considerable time and manpower of researchers. Currently, mucin-based media and modified BHI media are predominantly adopted for culturing *Akkermansia muciniphila* at home and abroad. These media entail high costs and require a culturing period of 3 to 5 days, which greatly restricts the large-scale production and application of this bacterium. Furthermore, the main components of mucin-based media and BHI media, i.e., brain heart infusion, are animal-derived products that carry potential risks, making them unsuitable for use in human food and pharmaceuticals.

Accordingly, there is a need in this field for a non-animal-derived culture medium that enables large-scale culturing of *Akkermansia muciniphila* with low cost, low nutrient loss, and high efficiency.

### Content of the Invention

In view of the above problems, the inventors of the present invention have for the first time applied a genome-scale metabolic network model to optimize the culture medium for *Akkermansia muciniphila*. By combining theoretical analysis with experiments, a low-cost, compositionally simple, non-animal-derived culture medium for *Akkermansia muciniphila* is finally developed, which enables high-density fermentation of the strain. The present invention is thus accomplished.

Accordingly, in a first aspect of the present disclosure, a culture medium for culturing *Akkermansia muciniphila* is provided, which includes:
a carbohydrate at a concentration of 2-200 g/L, where the carbohydrate is selected from disaccharide, oligosaccharide, polysaccharide, and any combination thereof;
a glucosamine and/or glucosamine polymer at a concentration of 2-200 g/L, where the glucosamine and/or glucosamine polymer is selected from D-glucosamine, D-glucosamine hydrochloride, N-acetyl-D-glucosamine, N-acetyl-D-galactosamine, N-acetyl-D-mannosamine, chitosan oligosaccharide, chitosan, chitin, and any combination thereof;
a non-animal-derived nitrogen source at a concentration of 5-200 g/L, where the non-animal-derived nitrogen source is selected from soybean peptone, wheat peptone, yeast extract, yeast peptone, and any combination thereof;
L-threonine, L-tryptophan, and L-phenylalanine at a concentration of 0.1-20 g/L;
a reducing agent at a concentration of 0-50 mM, where the reducing agent is selected from L-cysteine, L-cysteine hydrochloride, ascorbic acid, glutathione, SO₃²⁻, S₂O₃²⁻, S₂O₄²⁻, thiourea dioxide (CH₄N₂O₂S), and any combination thereof;
cobalamin at a concentration of 0-1 g/L;
Mg²⁺ at a concentration of 0-10 mM; and
a buffer salt at a concentration of 0-10 g/L, where the buffer salt is selected from dipotassium hydrogen phosphate, potassium dihydrogen phosphate, sodium dihydrogen phosphate, sodium bicarbonate, sodium carbonate, and any combination thereof.

In a second aspect, the present disclosure provides a method for culturing *Akkermansia muciniphila*, the method including culturing *Akkermansia muciniphila* in the culture medium described in the first aspect.

In summary, based on the genome-scale metabolic network model, the present invention obtains a set of metabolic uptake substances suitable for the growth of microorganisms, such as *Akkermansia muciniphila*, which serves as a reference for medium optimization. Components that promote microbial growth are further screened out from the metabolic uptake substances through experiments, thereby finally providing a high-density culture medium for *Akkermansia muciniphila*. This medium removes redundant components contained in conventional media. It features simple composition, low cost, and no animal-derived ingredients and can raise the viable count of *Akkermansia muciniphila* to more than 10¹⁰ cfu/mL, making the industrial production of *Akkermansia muciniphila* feasible. The culture medium of the present invention excludes various inorganic salts, especially Ca²⁺, thereby solving the problem of precipitate formation during high-temperature and high-pressure sterilization of conventional media and preventing damage to fermentation equipment. In addition, the medium contains no components prohibited for food and pharmaceutical production, such as Na₂S·9H₂O. It causes fewer Maillard reactions and less nutrient loss and accordingly supports favorable growth status, rapid proliferation, and a short culturing cycle of the bacteria. Meanwhile, it delivers excellent performance in downstream processing with a higher centrifugation recovery rate and almost no material loss. Furthermore, the non-animal-derived medium avoids contaminants and allergens, has a higher safety profile, and complies with the production requirements of the food and pharmaceutical industries.

### Description of the Drawings

To more clearly illustrate the embodiments of the present invention or the technical solutions in the prior art, the drawings required for describing the embodiments and the prior art are briefly introduced below.
FIG. 1 shows partial metabolic uptake substances obtained by simulating the normal growth of *Akkermansia muciniphila* via its genome-scale metabolic network model.
FIG. 2 shows the effects of different pH values on the high-density fermentation of *Akkermansia muciniphila*.
FIG. 3 shows the effects of fed-batch fermentation on the high-density fermentation of *Akkermansia muciniphila* when using the exemplary optimized culture medium of the present invention at inoculum sizes of 3×10⁷ cfu/mL, 6×10⁷ cfu/mL, and 1.2×10⁸ cfu/mL, where the group with an inoculum size of 1.2×10⁸ cfu/mL was subjected to feeding.
FIG. 4 presents the differences in downstream centrifugation between the control culture medium and the exemplary optimized culture medium of the present invention.
FIG. 5 shows the effects of *Akkermansia muciniphila* cells cultured with the control culture medium and the exemplary optimized culture medium of the present invention on the body weight of hyperlipidemic mice.
FIG. 6 is a collinearity comparison diagram of the genomic sequences of *Akkermansia muciniphila* cultured with the control culture medium and the exemplary optimized culture medium of the present invention (the upper graph shows the results of the control culture medium, and the lower graph shows the results of the exemplary optimized culture medium of the present invention).

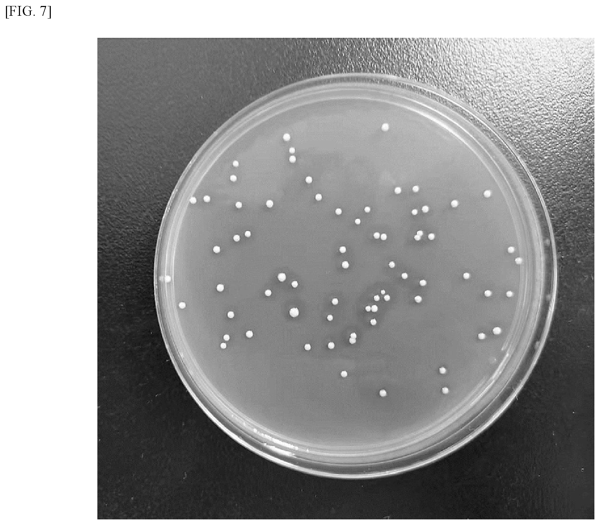

FIG. 7 shows single colonies of *Akkermansia muciniphila* cultured on a plate, which are prepared according to an embodiment of the present invention.

### Specific Implementations

The present invention will be described in detail below in conjunction with the drawings. It should be understood that the following description merely illustrates the present invention by way of examples and is not intended to limit its scope. The protection scope of the present invention shall be subject to the appended claims. Those skilled in the art will appreciate that various modifications may be made to the technical solutions of the present invention without departing from its spirit and essence. Unless otherwise specified, the technical means adopted in the examples are conventional means well known to those skilled in the art.

Unless otherwise defined, all technical and scientific terms used herein shall have the same meanings as commonly understood by those of ordinary skill in the art to which the present invention pertains. Prior to the detailed description of the present invention, the following definitions are provided for a better understanding thereof.

Where a numerical range is provided, such as a concentration range, a percentage range, or a ratio range, it should be understood that, unless the context clearly dictates otherwise, each intermediate value between the upper and lower limits of the range, down to one-tenth of the unit of the lower limit, as well as any other stated values or intermediate values within the range, is encompassed in the subject matter. The upper and lower limits of these smaller ranges may be independently included in the smaller ranges, and such embodiments are also encompassed in the subject matter, subject to any specifically excluded limit value in the range. Where the range includes one or two limit values, ranges excluding either or both of those included limit values are also encompassed in the subject matter.

In the context of the present invention, many embodiments adopt the expressions "comprising," "including," or "consisting essentially of." The expressions "comprising," "including," and "consisting essentially of" are generally construed as open-ended terms, which mean that the subject matter covers not only the specifically listed elements, components, assemblies, and method steps, but also other unlisted ones. In addition, in certain cases, these expressions may also be interpreted as closed-ended terms, which mean that the subject matter consists solely of the specifically listed elements, components, assemblies, and method steps, with no other elements, components, assemblies, or method steps included. In such cases, these expressions are equivalent to "consisting of."

As mentioned above, based on the genome-scale metabolic network model, the present invention obtains a set of metabolic uptake substances suitable for the growth of microorganisms such as *Akkermansia muciniphila*, which serves as a reference for medium optimization. Components that promote microbial growth are further screened out from the set of metabolic uptake substances through experiments, thereby finally providing a high-density culture medium for *Akkermansia muciniphila*.

Accordingly, in a first aspect of the present disclosure, a culture medium for culturing *Akkermansia muciniphila* is provided, which includes:
a carbohydrate at a concentration of 2-200 g/L, where the carbohydrate is selected from disaccharide, oligosaccharide, polysaccharide, and any combination thereof;
a glucosamine and/or glucosamine polymer at a concentration of 2-200 g/L, where the glucosamine and/or glucosamine polymer is selected from D-glucosamine, D-glucosamine hydrochloride, N-acetyl-D-glucosamine, N-acetyl-D-galactosamine, N-acetyl-D-mannosamine, chitosan oligosaccharide, chitosan, chitin, and any combination thereof;
a non-animal-derived nitrogen source at a concentration of 5-200 g/L, where the non-animal-derived nitrogen source is selected from soybean peptone, wheat peptone, yeast extract, yeast peptone, and any combination thereof;
L-threonine, L-phenylalanine, and L-tryptophan at a concentration of 0.1-10 g/L;
a reducing agent at a concentration of 0-50 mM, where the reducing agent is selected from L-cysteine, L-cysteine hydrochloride, ascorbic acid, reduced glutathione, SO₃²⁻, S₂O₃²⁻, S₂O₄²⁻, thiourea dioxide (CH₄N₂O₂S), and any combination thereof;
cobalamin at a concentration of 0-1 g/L;
Mg²⁺ at a concentration of 0-10 mM; and
a buffer salt at a concentration of 0-10 g/L, where the buffer salt is selected from dipotassium hydrogen phosphate, potassium dihydrogen phosphate, sodium dihydrogen phosphate, sodium bicarbonate, sodium carbonate, and any combination thereof.

In the context of the present disclosure, those skilled in the art will recognize that both carbohydrate and glucosamine and/or glucosamine polymer can serve as carbon sources for culturing *Akkermansia muciniphila*.

In a preferred embodiment, the concentration of the carbohydrate is 2, 3, 4, 5, 6, 7, 8, 9, 10, 15, 20, 30, 40, 50, 100, 150, or 200 g/L, or any value therebetween. In a preferred embodiment, the concentration of the carbohydrate is 2-50 g/L. In a more preferred embodiment, the concentration of the carbohydrate is 4-20 g/L.

In a specific embodiment, the disaccharide includes, but is not limited to, lactose, maltose, isomaltulose, cellobiose, turanose, melibiose, and any combination thereof.

In another specific embodiment, the oligosaccharide includes, but is not limited to, galacto-oligosaccharide, fructo-oligosaccharide, mannan-oligosaccharide, isomalto-oligosaccharide, malto-oligosaccharide, soybean oligosaccharide, human milk oligosaccharide, and any combination thereof.

In a further specific embodiment, the human milk oligosaccharide includes, but is not limited to, 2'-fucosyllactose, 3'-sialyllactose, lacto-N-neotetraose, lacto-N-triose, and any combination thereof.

In another specific embodiment, the polysaccharide includes, but is not limited to, dextrin, starch, modified starch, dextran, and any combination thereof. The dextrin includes maltodextrin, resistant dextrin, cyclodextrin, and the like. The modified starch includes pregelatinized starch, soluble starch, and the like.

In a preferred embodiment, the concentration of the glucosamine and/or glucosamine polymer is 2, 3, 4, 5, 6, 7, 8, 9, 10, 15, 20, 30, 40, 50, 100, 150, or 200 g/L, or any value therebetween. In a preferred embodiment, the concentration of the glucosamine and/or glucosamine polymer is 2-50 g/L. In a more preferred embodiment, the concentration of the glucosamine and/or glucosamine polymer is 5-20 g/L.

In another preferred embodiment, the glucosamine and/or glucosamine polymer includes, but is not limited to, N-acetyl-D-glucosamine, N-acetyl-D-mannosamine, chitosan, and any combination thereof.

In a preferred embodiment, the non-animal-derived nitrogen source is yeast extract.

The concentration of the non-animal-derived nitrogen source is 5, 6, 7, 8, 9, 10, 15, 20, 30, 40, 50, 100, 150, or 200 g/L, or any value therebetween. In a preferred embodiment, the concentration of the non-animal-derived nitrogen source is 5-50 g/L.

In a more preferred embodiment, the concentration of the non-animal-derived nitrogen source is 10-20 g/L.

In the context of the present disclosure, those skilled in the art will appreciate that, apart from the nitrogen sources listed above, glucosamine and/or glucosamine polymer can also serve as nitrogen sources for culturing *Akkermansia muciniphila*.

In a preferred embodiment, the concentration of L-threonine is 0.1, 0.2, 0.3, 0.4, 0.5, 1, 2, 3, 4, 5, 6, 7, 8, 9, or 10 g/L, or any value therebetween. In a preferred embodiment, the concentration of L-threonine is 2-10 g/L. In a more preferred embodiment, the concentration of L-threonine is 4-8 g/L.

In a further preferred embodiment, the culture medium further includes other amino acids in addition to L-threonine. The other amino acids are selected from L-phenylalanine, L-tryptophan, and any combination thereof. The inventors have found that compared with other amino acids, L-threonine added to the culture medium can more significantly promote the growth of *Akkermansia muciniphila*. Moreover, the additional supplementation of L-phenylalanine and/or L-tryptophan on the basis of L-threonine can further significantly promote the growth of the strain.

Accordingly, in a more preferred embodiment, the amino acids are a combination of L-threonine and L-phenylalanine, a combination of L-threonine and L-tryptophan, or a combination of L-threonine, L-phenylalanine, and L-tryptophan.

In the context of the present disclosure, the concentration of the other amino acids is 0, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, or 2.0 g/L, or any value therebetween. In a preferred embodiment, the concentration of the other amino acids is 0.5-2.0 g/L.

In a preferred embodiment, the concentration of cobalamin is 0, 1, 2, 3, 4, 5, 6, 7, 8, 9, 10, 20, 30, 40, 50, 75, 100, 150, 200, 350, 500, 800, or 1000 mg/L, or any value therebetween. In a preferred embodiment, the concentration of cobalamin is 0.1-10 mg/L. The inventors have discovered that supplementation with only cobalamin can significantly boost the growth of *Akkermansia muciniphila*, in contrast to other vitamins. Therefore, the culture medium of the present invention may contain only cobalamin without any other vitamins.

In another preferred embodiment, the concentration of the reducing agent is 0, 1, 2, 3, 4, 5, 6, 7, 8, 9, 10, 20, 30, 40, or 50 mM, or any value therebetween. In a preferred embodiment, the concentration of the reducing agent is 0.1-10 mM. In a more preferred embodiment, the concentration of the reducing agent is 1-10 mM.

The inventors have found that *Akkermansia muciniphila* almost does not grow in a culture medium without a reducing agent. Commonly used Na₂S and its hydrates, such as Na₂S·9H₂O are prohibited for use in food and pharmaceutical production. Hence, it is necessary to develop a reducing agent that can replace Na₂S and its hydrates. Reducing agents widely applied in probiotic culturing, including ascorbic acid, reduced glutathione, and L-cysteine hydrochloride, as well as S₂O₃²⁻ (e.g., Na₂S₂O₃·5H₂O), S₂O₃²⁻, S₂O₄²⁻, and thiourea dioxide (CH₄N₂O₂S), achieve a reducing effect equivalent to that of Na₂S·9H₂O within the selected concentration range and can serve as a drop-in substitute for Na₂S·9H₂O.

In a preferred embodiment, the reducing agent is S₂O₃²⁻.

In addition, it is understood that in the present invention, S₂O₃²⁻ and S₂O₄²⁻ can be derived from their sodium salts, potassium salts, or hydrates, such as Na₂S₂O₃·5H₂O and Na₂S₂O₄, but are not limited thereto.

The concentration of Mg²⁺ is 0, 1, 2, 3, 4, 5, 6, 7, 8, 9, or 10 mM, or any value therebetween. In a preferred embodiment, the concentration of Mg²⁺ ranges from 0 to 5 mM. It is understood that in the present invention, Mg²⁺ is derived from magnesium chloride, magnesium sulfate, or hydrates thereof, but is not limited thereto.

The inventors of the present invention have found that Ca²⁺, an inorganic ion commonly used in culture media for *Akkermansia muciniphila*, has a negligible effect on the growth of the strain. Therefore, Ca²⁺ may be omitted from the culture medium, which avoids precipitate formation under high-temperature and high-pressure sterilization and further prevents damage to culture equipment.

In addition, the inventors of the present invention have also discovered that the acidic trace element solutions (e.g., FeCl₂, H₃BO₃, ZnCl₂, CuCl₂·2H₂O, MnCl₂, CoCl₂, NiCl₂) and alkaline trace element solutions (e.g., Na₂SeO₃, Na₂WO₄·2H₂O, Na₂MoO₄·2H₂O) conventionally used for culturing *Akkermansia muciniphila* exert negligible influence on its growth, so they are not required in the culture medium.

In another specific embodiment, the fermentation medium further includes a buffer salt at a concentration of 2 to 7 g/L.

In another specific embodiment, the concentration of potassium dihydrogen phosphate, disodium hydrogen phosphate, and dipotassium hydrogen phosphate each ranges from 0 to 4.5 g/L. In a preferred embodiment, the concentration of potassium dihydrogen phosphate, disodium hydrogen phosphate, and dipotassium hydrogen phosphate each ranges from 0 to 2 g/L.

In another specific embodiment, the concentration of sodium bicarbonate ranges from 1 to 10 g/L. In a preferred embodiment, the concentration of sodium bicarbonate ranges from 1 to 5 g/L.

In a more preferred embodiment, the culture medium includes: 4-15 g/L carbohydrate, 10-20 g/L glucosamine and/or glucosamine polymer, 10-20 g/L yeast extract, 1-1.2 g/L phenylalanine, 0.25 g/L sodium thiosulfate pentahydrate or an equivalent amount of sodium thiosulfate or 0.11 g/L CH₄N₂O₂S, 4-8 g/L L-threonine, and 0.1 mg/L cobalamin; and optionally, 0.5 g/L tryptophan, 0.1-0.12 g/L magnesium chloride, and 6-7 g/L buffer salt.

Without being bound by theory, those skilled in the art will appreciate that the culture medium of the present invention is applicable to both high-density culture and static culture of *Akkermansia muciniphila*.

As commonly used in the art, the term "static culture" refers to a cell culture technique performed in a container filled with liquid culture medium without aeration or agitation.

As commonly used in the art, the term "high-density culture," also known as high-density fermentation, refers to a culture technique where the cell population density of microorganisms in liquid culture is more than 10 times that of conventional culture.

As commonly used in the art, high-density culture can be carried out in a fed-batch fermentation mode, for example, with continuous feeding during the culture process. As commonly used in the art, the term "fed-batch fermentation" refers to a fermentation technique in which, during a batch fermentation process of microorganisms, certain materials are added to the fermentation system in some manner, while the fermentation broth is not continuously withdrawn. This fermentation technique is an intermediate between batch fermentation and continuous fermentation. Depending on whether the feeding is intermittent or continuous, it can be divided into intermittent feeding and continuous feeding. Those skilled in the art will appreciate that a high concentration of substrates in the culture medium may inhibit microbial growth. In fed-batch fermentation, the fermentation is initially carried out at a relatively low nutrient concentration, followed by continuous addition of fresh nutrients, thereby allowing normal microbial growth and continuous accumulation of metabolites and avoiding substrate inhibition caused by excessive one-time feeding in batch fermentation.

As commonly used in the art, the feed may be a complete feeding medium or a semi-fed-batch medium. The "complete feeding medium" refers to a medium containing complete nutritional components. The "semi-fed-batch medium" refers to an incomplete medium containing only one or several nutritional components, such as an incomplete medium containing only carbon sources (e.g., maltose, maltodextrin, N-acetylglucosamine, lactose, N-acetylmannosamine, and any combination thereof); or an incomplete medium containing carbon sources (e.g., maltose, maltodextrin, N-acetylglucosamine, lactose, N-acetylmannosamine, and any combination thereof) and nitrogen sources (e.g., soybean peptone, wheat peptone, yeast extract, yeast peptone, and any combination thereof); or a medium at several-fold concentration (e.g., 1- to 10-fold) without a buffer solution.

In a second aspect of the present disclosure, a method for culturing *Akkermansia muciniphila* is provided. The method includes culturing *Akkermansia muciniphila* in the culture medium according to the first aspect.

As stated above and without being bound by theory, the culture medium of the present invention is applicable to both high-density culture and static culture.

In a specific embodiment, the culture is static culture, high-density culture, or static culture followed by high-density culture.

In another specific embodiment, the culture is performed under anaerobic conditions. The "anaerobic conditions" are well known to those skilled in the art, for example, a gas mixture containing 5-10% H₂, 5-10% CO₂, and 80-85% N₂.

In another specific embodiment, the static culture includes culturing at 36 °C-38 °C for 12-36 h with 2-3 passages. In a preferred embodiment, the 2nd and 3rd passages are carried out using the culture medium according to the second aspect.

In another specific embodiment, for the high-density culture, *Akkermansia muciniphila* is inoculated into the culture medium at an inoculum size of 3×10⁷ cfu/mL to 3×10⁸ cfu/mL.

In a preferred embodiment, *Akkermansia muciniphila* is inoculated into the culture medium at an inoculum size of 3×10⁷ cfu/mL to 1.5×10⁸ cfu/mL.

In a more preferred embodiment, *Akkermansia muciniphila* is inoculated into the culture medium at an inoculum size of 3×10⁷ cfu/mL to 1.2×10⁸ cfu/mL.

In another specific embodiment, the high-density culture is performed at a temperature of 20 °C to 40 °C. In a preferred embodiment, the high-density culture is performed at a temperature of 36 °C to 38 °C. In a further preferred embodiment, the high-density culture is performed at 37 °C.

In another specific embodiment, the high-density culture is carried out for 24 h to 36 h.

In another specific embodiment, the pH of the culture medium is controlled within a range of 6 to 8 during high-density culture. In a preferred embodiment, the pH of the culture medium is controlled within a range of 6.0 to 7.5.

In another specific embodiment, the high-density culture is conducted at a rotation speed of 50 to 300 rpm. In a preferred embodiment, the high-density culture is conducted at a rotation speed of 50 to 150 rpm.

Without being bound by theory, a culture medium different from that for high-density culture may be used for static culture prior to high-density culture. In an exemplary embodiment, the culture medium for the preceding static culture includes, but is not limited to, BHI medium, mucin medium, and Columbia medium.

In another specific embodiment, the high-density culture is carried out in a fed-batch fermentation mode; for example, continuous feeding is applied during the culture process.

In a further specific embodiment, the feed is a complete feeding medium or a semi-fed-batch medium. As described above, the "complete feeding medium" refers to a medium containing complete nutritional components. The "semi-fed-batch medium" refers to an incomplete medium containing only one or several nutritional components, such as an incomplete medium containing only carbon sources (e.g., maltose, maltodextrin, D-glucosamine, N-acetylglucosamine, lactose, N-acetylmannosamine, and any combination thereof); or an incomplete medium containing carbon sources and nitrogen sources (e.g., soybean peptone, wheat peptone, yeast extract, yeast peptone, and any combination thereof); or a medium at 1- to 10-fold concentration (e.g., 3- to 5-fold) without a buffer solution.

In a specific embodiment, the feed is a semi-fed-batch medium. By way of example, the semi-fed-batch medium includes, but is not limited to, carbon sources at 1- to 10-fold concentration, such as carbon sources with a concentration of 240 g/L. The carbon sources are selected from maltose, maltodextrin, D-glucosamine, N-acetylglucosamine, lactose, N-acetylmannosamine, and any combination thereof.

In a preferred embodiment, continuous feeding is performed at a feeding rate of 1 mL·L⁻¹·h⁻¹ to 6 mL·L⁻¹·h⁻¹ during high-density culture, where mL represents the volume of the feeding medium, L represents the volume of the original fermentation system, and h represents the feeding duration. In a further preferred embodiment, continuous feeding is performed at a feeding rate of 1.2 mL·L⁻¹·h⁻¹ during high-density culture.

Those skilled in the art will appreciate that based on the culture medium for *Akkermansia muciniphila* optimized by the method of the present invention, supplementing one or more nutrients required for fermentation of the strain (such as carbon sources) enables continuous and massive growth and reproduction of the bacteria. This realizes a high-density culture of *Akkermansia muciniphila* and avoids the decrease in bacterial biomass caused by prolonged centrifugation after fermentation. Furthermore, the fed-batch process can further reduce fermentation time, shorten the fermentation cycle, improve fermentation efficiency, simplify operating procedures, lower equipment investment, and cut production costs. This is highly favorable for large-scale production of *Akkermansia muciniphila*.

In a third aspect of the present disclosure, provided is a strain Akk-101 of *Akkermansia muciniphila* obtained by the method according to the second aspect of the present disclosure. The strain was deposited on September 13, 2023, at the China General Microbiological Culture Collection Center (CGMCC), located at the Institute of Microbiology, Chinese Academy of Sciences, No. 3, Yard 1, Beichen West Road, Chaoyang District, Beijing, China, with the deposit accession number CGMCC NO. 40786.

In other aspects of the present disclosure, provided is a method for optimizing microbial culture media based on a genome-scale metabolic network model, which includes the following steps:
1) Constructing a genome-scale metabolic network model based on all protein sequences of the microbial genome. The genome-scale metabolic network model covers biochemical reactions occurring in three compartments of microbial cells, namely the extracellular space, periplasm, and cytoplasm, and the biochemical reactions include a simulated bacterial growth reaction.
2) Based on the genome-scale metabolic network model, performing flux balance analysis multiple times under different uptake substrate conditions composed of various uptake substances involved in the biochemical reactions to simulate the normal growth of the microorganism, thereby obtaining a set of metabolic uptake substances required for the normal growth of the microorganism.
3) For each metabolic uptake substance in the set of metabolic uptake substances, comparing the growth of the microorganism in reference culture media with and without the metabolic uptake substance to determine its effect on microbial growth, thereby obtaining metabolic uptake substances that promote microbial growth, which are the candidate components for the microbial culture medium.

In the context of the present disclosure, the term "metabolic network model" refers to a genome scale of metabolic model (GSMM), which contains most biochemical reactions taking place in a given microbial cell (e.g., bacteria). Metabolic regulation in microbial cells occurs at multiple levels, including gene-gene, gene-protein, protein-protein, protein-metabolite, and physical interactions. Integrated with other omics data such as transcriptomics and proteomics data, GSMM enables an in-depth understanding of the regulatory mechanisms inside microbial cells. Therefore, GSMM serves as an efficient platform to interpret the physiological and metabolic functions of microorganisms from a global perspective and is widely used for qualitative and quantitative prediction of cellular growth phenotypes under different metabolic or environmental perturbations. Flux Balance Analysis (FBA) is a mathematical method developed in recent years for constructing, simulating, and analyzing GSMM.

In a specific embodiment, the method further includes classifying the candidate culture medium components and simplifying the components of the microbial culture medium on the basis of the classification results.

Those skilled in the art will appreciate that the medium optimization method of the present invention is universal and applicable to a variety of bacterial genera, including but not limited to *Akkermansia*, *Lactobacillus*, *Blautia*, and *Bifidobacterium*. In the context of the present disclosure, the inventors adopted the above method to optimize the culture medium for *Akkermansia muciniphila* of the genus *Akkermansia* and, accordingly, developed a high-density culture medium and a corresponding culture process for this strain.

Hereinafter, the present invention will be described in further detail in conjunction with exemplary embodiments. However, the exemplary embodiments disclosed herein are provided for illustrative purposes only and shall not be construed as limitations on the scope of the present invention.

### Examples

The following examples illustrate the method for optimizing the culture medium of *Akkermansia muciniphila* by using the metabolic network model analysis method of the present invention, as well as the optimized culture medium, high-density culture method, and relevant characterization results of the strain. Unless otherwise specified, all experimental methods used herein are conventional techniques, and all experimental materials are commercially available from conventional reagent suppliers. Unless otherwise defined, all technical and scientific terms used herein have the same meanings as commonly understood by those skilled in the technical field to which the present invention pertains.

It should be noted that the terms used in the specification of the present invention are merely for describing specific embodiments and are not intended to limit the present invention. The foregoing summary of the invention and the following detailed description are only intended to elaborate on the present invention and shall not be construed as limiting the present invention in any manner. Without departing from the spirit and scope of the present invention, the scope of the present invention shall be defined by the appended claims.

### Example 1: Acquisition of Candidate Medium Components Using the Genome-Scale Metabolic Network Model of Akkermansia muciniphila

Step 1: All protein sequences of the *Akkermansia muciniphila* genome are retrieved from NCBI. The protein sequence file of *Akkermansia muciniphila* is named GCF_000020225.1_ASM2022v1_protein.faa.gz, which is stored at the following path: https://ftp.ncbi.nlm.nih.gov/genomes/all/GCF/000/020/225/GCF_000020225.1_ASM2022v1/.

Step 2: A genome-scale metabolic network model is constructed using the CarveMe software (available at https://github.com/cdanielmachado/carveme) on the basis of the obtained protein sequences of the *Akkermansia muciniphila* genome. The procedures for constructing the genome-scale metabolic network model are described in the guidelines published at https://carveme.readthedocs.io/en/latest/. The generated metabolic network model file is saved in SBML format (see http://sbml.org/).

Substance flows in three compartments of bacterial cells, namely the extracellular space, periplasm, and cytosol, are simulated by the established genome-scale metabolic network model of *Akkermansia muciniphila*. A total of 737 compounds and 1490 biochemical reactions are involved in the model, which are categorized as follows:
904 enzyme-catalyzed biochemical reactions
431 transport reactions for simulating the translocation of compounds among different compartments of bacterial cells
151 exchange reactions for simulating bacterial uptake and secretion of compounds
1 reaction for simulating bacterial growth
1 ATP synthesis reaction for simulating cellular energy maintenance
2 dummy reactions for simulating the absorption and generation of intracellular compounds

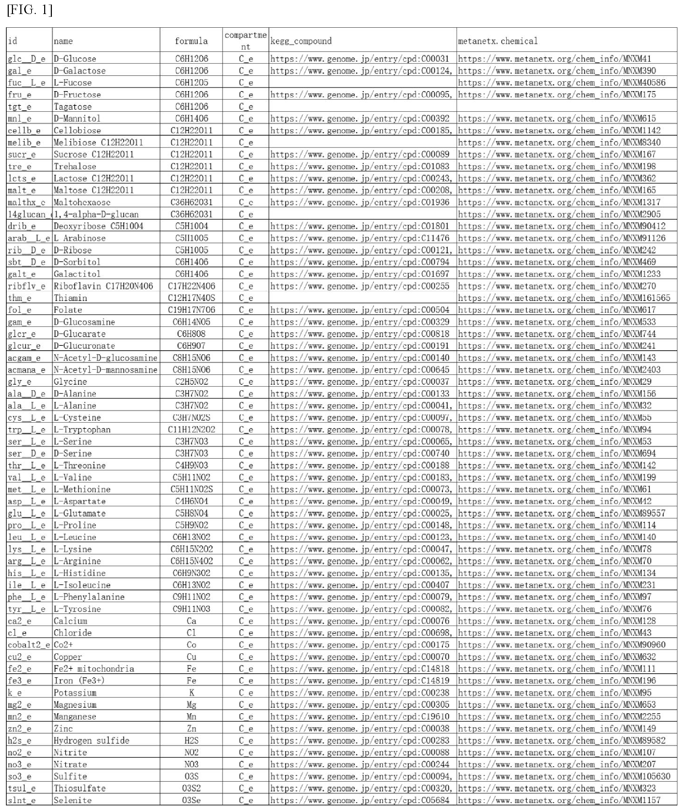

Step 3: Multiple simulations of the normal growth of *Akkermansia muciniphila* under different nutritional conditions are performed by using the genome-scale metabolic network model. A variety of potential uptake substances and secreted substances are obtained for subsequent experimental analysis. The specific uptake and secreted substances are presented in FIG. 1.

### Example 2: Effects of Different Carbohydrates on the Growth of Akkermansia muciniphila

A control medium was prepared according to the formula reported by Plovier H et al., and the composition was detailed as follows: 0.4 g/L KH₂PO₄, 0.53 g/L Na₂HPO₄, 0.3 g/L NH₄Cl, 0.3 g/L NaCl, 0.1 g/L MgCl₂, 0.11 g/L CaCl₂, 4 g/L NaHCO₃, 0.25 g/L Na₂S·9H₂O, 0.5 mg/L resazurin, 1 mL/L acidic trace element solution, 1 mL/L alkaline trace element solution, 1 mL/L vitamin solution, 16 g/L soybean peptone, 4 g/L threonine, 4.5 g/L glucose, and 5.5 g/L N-acetylglucosamine. The composition of the acidic trace element solution was as follows: 0.95 g/L FeCl₂, 0.62 g/L H₃BO₃, 0.068 g/L ZnCl₂, 0.017 g/L CuCl₂·2H₂O, 0.063 g/L MnCl₂, 0.065 g/L CoCl₂, 0.013 g/L NiCl₂, and 50 mmol/L HCl. The composition of the alkaline trace element solution was as follows: 0.017 g/L Na₂SeO₃, 0.033 g/L Na₂WO₄·2H₂O, 0.024 g/L Na₂MoO₄·2H₂O, and 0.4 g/L NaOH. The composition of the vitamin solution was as follows: 0.2 g/L thiamine, 0.1 g/L riboflavin, 0.2 g/L nicotinic acid, 0.1 g/L calcium pantothenate, 0.5 g/L pyridoxine, 0.2 g/L biotin, 0.1 g/L cobalamin, and 0.1 g/L p-aminobenzoic acid.

On the basis of the above control medium, glucose in the control medium was replaced with carbohydrates or sugar alcohols listed in Table 1 at a uniform concentration of 4.5 g/L. After culture, the OD₆₀₀ values were compared with those of the control medium to evaluate the effects of each candidate medium component on the growth of *Akkermansia muciniphila.*

The specific experimental procedures were as follows: The preserved strain was removed from a -80 °C freezer and pre-cultured in the control medium for 24 h. Subsequently, the bacterial suspension was inoculated at an inoculum size of 1%. Culture was carried out under anaerobic conditions (10% H₂, 10% CO₂, and 80% N₂) for 36 h. The OD₆₀₀ value was then determined using a microplate reader (INFINITE M NANO, Tecan, Switzerland) with a loading volume of 200 µL per well. The results are shown in Table 1.

**Table 1: Effects of Candidate Carbohydrates and Sugar Alcohols on the Growth of Akkermansia muciniphila**

| No. | Carbohydrates | OD₆₀₀ | No. | Carbohydrates | OD₆₀₀ |
|---|---|---|---|---|---|
| 1 | Glucose (control) | 0.7647 | 9 | Isomaltulose (disaccharide) | 0.8174** |
| 2 | Mannose (monosaccharide) | 0.7851 | 10 | Cellobiose (disaccharide) | 0.8235** |
| 3 | Fucose (monosaccharide) | 0.5984 ** | 11 | Fructooligosaccharide (oligosaccharide) | 0.8226** |
| 4 | Galactose (monosaccharide) | 0.6715^{**} | 12 | Galactooligosaccharide (oligosaccharide) | 0.8304^{**} |
| 5 | Fructose (monosaccharide) | 0.7792 | 13 | Isomaltooligosaccharide (oligosaccharide) | 0.8425^{**} |
| 6 | Lactulose (disaccharide) | 0.7769 | 14 | Maltodextrin (polysaccharide) | 0.8406^{**} |
| 7 | Lactose (disaccharide) | 0.8133^{**} | 15 | Dextran (polysaccharide) | 0.8660^{**} |
| 8 | Maltose (disaccharide) | 0.8662^{**} | 16 | Soluble starch (polysaccharide) | 0.8338^{**} |

| | | | | | |
|---|---|---|---|---|---|
| Note: * indicates p < 0.05 versus the control group; ** indicates p < 0.01 versus the control group. | | | | | |

It can be seen from Table 1 that none of the monosaccharides produced a significant increase in OD₆₀₀ compared with the control group. On the contrary, fucose and galactose triggered intense Maillard reactions, resulting in a pronounced decrease in OD₆₀₀. Among disaccharides, all varieties except lactulose markedly increased OD₆₀₀, indicating that these disaccharides could better support the growth of *Akkermansia muciniphila* than glucose. Only six types of oligosaccharides and polysaccharides were tested in this example, namely fructooligosaccharide, galactooligosaccharide, isomaltooligosaccharide, maltodextrin, soluble starch, and dextran. All of them caused a highly significant rise in OD₆₀₀, which demonstrates that they can greatly promote the growth of *Akkermansia muciniphila* in comparison with glucose.

### Example 3: Effects of Different Reducing Agents on the Growth of Akkermansia muciniphila

The control medium was prepared according to the formula reported by Plovier H et al. in Example 2, in which Na₂S·9H₂O at a concentration of 0.25 g/L (1 mM) was used as the reducing agent. The Na₂S·9H₂O was separately replaced with Na₂S₂O₃·5H₂O, Na₂S₂O₄, CH₄N₂O₂S, Na₂SO₃, L-cysteine hydrochloride, ascorbic acid, and reduced glutathione to prepare culture media containing different reducing agents for comparative analysis with the control medium.

The specific experimental procedures were as follows: The preserved strain was removed from a -80 °C freezer and pre-cultured in the above control medium for 24 h. Subsequently, the bacterial suspension was inoculated at an inoculum size of 1%. Culture was carried out under anaerobic conditions (10% H₂, 10% CO₂, and 80% N₂) for 36 h. The OD₆₀₀ value was then determined using a microplate reader (INFINITE M NANO, Tecan, Switzerland) with a loading volume of 200 µL per well. The results are shown in Table 2.

**Table 2: Effects of Different Reducing Agents on the Growth of Akkermansia muciniphila**

| Test No. | | Type of Reducing Agent | Dosage | OD₆₀₀ |
|---|---|---|---|---|
| 1 | Control group | Na₂S·9H₂O | 1 mM | 0.7633 |
| 2 | Blank group | No reducing agent | / | 0.3178** |
| 3 | Experimental group | Na₂S₂O₃·5H₂O | 1 mM | **0.7686** |
| 4 | | Na₂S₂O₄ | 1 mM | **0.7584** |
| 5 | | CH₄N₂O₂S | 1 mM | **0.7563** |
| 6 | | Na₂SO₃ | 1 mM | 0.5529** |
| 7 | | L-cysteine hydrochloride | 1 mM | 0.5026** |
| 8 | | | 5 mM | 0.6755** |
| 9 | | Ascorbic acid | 1 mM | 0.3657** |
| 10 | | | 5 mM | 0.5528** |
| 11 | | | 10 mM | 0.6843** |
| 12 | | Reduced glutathione | 1 mM | 0.3966** |
| 13 | | | 3 mM | 0.5901** |

| | | | | |
|---|---|---|---|---|
| Note: * indicates p < 0.05 versus the control group; ** indicates p < 0.01 versus the control group. | | | | |

It can be seen from the results in Table 2 that *Akkermansia muciniphila* barely grows in the medium without any reducing agent (blank group). The experimental groups supplemented with Na₂S₂O₃·5H₂O, Na₂S₂O₄, and CH₄N₂O₂S showed no significant differences in OD₆₀₀ values compared with the control group. Na₂S·9H₂O is prohibited for use in food and pharmaceutical production, while Na₂S₂O₃·5H₂O, Na₂S₂O₄, and CH₄N₂O₂S are widely used in food and pharmaceutical production, so they can serve as alternatives to the original reducing agent in the medium. Although Na₂SO₃ is permitted for food and pharmaceutical use, its performance is inferior to that of Na₂S·9H₂O. Ascorbic acid, reduced glutathione, and L-cysteine hydrochloride are commonly used in probiotic cultivation, yet their reducing effects are also weaker than Na₂S·9H₂O. According to the flux balance analysis in the present invention, S₂O₃²⁻(Na₂S₂O₃·5H₂O) can completely replace Na₂S·9H₂O with equivalent efficacy.

### Example 4: Effects of Different Amino Acids on the Growth of Akkermansia muciniphila

The control medium was prepared according to the formula reported by Plovier H et al. in Example 2, which contained 4 g/L threonine. On the basis of the control medium, different amino acids listed in Table 3 were separately added at a concentration of 0.5 g/L to prepare media supplemented with different amino acids for comparative analysis with the control medium.

The specific experimental procedures were as follows: The preserved strain was removed from a -80 °C freezer and pre-cultured in the control medium for 24 h. Subsequently, the bacterial suspension was inoculated at an inoculum size of 1%. Culture was carried out under anaerobic conditions (10% H₂, 10% CO₂, and 80% N₂) for 36 h. The OD₆₀₀ value was then determined using a microplate reader (INFINITE M NANO, Tecan, Switzerland) with a loading volume of 200 µL per well. The results are shown in Table 3.

**Table 3: Effects of Different Amino Acids on the Growth of Akkermansia muciniphila**

| **Amino Acid** | **OD₆₀₀** |
|---|---|
| **Threonine (control)** | **0.7715** |
| **Glycine** | 0.7609 |
| **Alanine** | 0.7807 |
| **Cysteine** | 0.7712 |
| **Tryptophan** | **0.8309**^{**} |
| **Serine** | 0.7717 |
| **Valine** | 0.7668 |
| **Methionine** | 0.7642 |
| **Aspartic acid** | 0.7643 |
| **Glutamic acid** | 0.7649 |
| **Proline** | 0.7836 |
| **Leucine** | 0.7872 |
| **Lysine** | 0.7679 |
| **Arginine** | 0.7615 |
| **Histidine** | 0.7772 |
| **Isoleucine** | 0.7797 |
| **Phenylalanine** | **0.8451^{**}** |
| **Tyrosine** | 0.7787 |

| | |
|---|---|
| Note: * indicates p < 0.05 versus the control group; ** indicates p < 0.01 versus the control group. | |

It can be seen from the results in Table 3 that among all tested amino acids, additional supplementation with tryptophan or phenylalanine can promote the growth of *Akkermansia muciniphila*. Cysteine and L-cysteine hydrochloride are commonly used reducing agents for probiotic culture and can facilitate the growth of anaerobic bacteria. However, they showed no growth-promoting effect on *Akkermansia muciniphila* in this medium. It is speculated that Na₂S·9H₂O in the original medium provided sufficient reducing power, so extra cysteine was not required. According to the flux balance analysis conducted in the present invention, the addition of phenylalanine and tryptophan is capable of boosting the growth of *Akkermansia muciniphila*.

### Example 5: Effects of Different Vitamins on the Growth of Akkermansia muciniphila

The control medium was prepared according to the formula reported by Plovier H et al. in Example 2, to which 1 mL of vitamin solution was added. The final concentrations of individual vitamins in the vitamin solution were as follows: thiamine 0.2 mg/L, riboflavin 0.1 mg/L, nicotinic acid 0.2 mg/L, calcium pantothenate 0.1 mg/L, pyridoxine 0.5 mg/L, biotin 0.2 mg/L, cobalamin 0.1 mg/L and p-aminobenzoic acid 0.1 mg/L. A blank group medium without any vitamins and experimental group media supplemented with a single type of vitamin were prepared separately. In addition, two experimental group media containing 1 mg/L hemin and 5 µg/L folic acid were also prepared.

**Table 4: Effects of Different Vitamins on the Growth of Akkermansia muciniphila**

| **Test No.** | **Group** | **Type of Vitamin** | **OD₆₀₀** |
|---|---|---|---|
| 1 | Control group | Vitamin solution | **0.8286**^{**} |
| 2 | Blank group | No vitamin | 0.7022 |
| 3 | Experimental group | Thiamine | 0.6967 |
| 4 | | Riboflavin | 0.6892 |
| 5 | | Nicotinic acid | 0.7050 |
| 6 | | Calcium pantothenate | 0.7064 |
| 7 | | Pyridoxine | 0.6929 |
| 8 | | Biotin | 0.7020 |
| 9 | | Cobalamin | **0.8257^{**}** |
| 10 | | p-Aminobenzoic acid | 0.7069 |
| 11 | | Hemin | 0.6922 |
| 12 | | Folic acid | 0.7018 |

| | | | |
|---|---|---|---|
| Note: * indicates p < 0.05 versus the control group; ** indicates p < 0.01 versus the control group. | | | |

It can be seen from the results in Table 4 that the OD₆₀₀ value of the control group was significantly higher than that of the blank group, indicating that the vitamin solution used in the control group could promote the growth of *Akkermansia muciniphila*. When individual vitamins were added separately, only cobalamin produced a statistically significant difference in OD₆₀₀ compared with the blank group. This demonstrates that among all components in the vitamin solution, only cobalamin exerts a growth-promoting effect on *Akkermansia muciniphila*. All other vitamins showed no significant differences relative to the blank group, meaning they had no positive effect on the growth of this strain. Likewise, hemin and folic acid failed to promote the growth of *Akkermansia muciniphila*.

### Example 6: Comparison Between Optimized Medium and Control Medium

The control medium was prepared according to the formula reported by Plovier H et al. in Example 2, and the OD₆₀₀ values of the optimized medium and the control medium were compared.

The specific experimental procedures were as follows: The preserved strain was removed from a -80 °C freezer and pre-cultured in the control medium for 24 h. Subsequently, the bacterial suspension was inoculated at an inoculum size of 1%. Culture was carried out under anaerobic conditions (10% H₂, 10% CO₂, and 80% N₂) for 36 h in the control medium and the optimized medium separately. The OD₆₀₀ value was then determined using a microplate reader (INFINITE M NANO, Tecan, Switzerland) with a loading volume of 200 µL per well. The results are shown in Table 5.

It can be seen from the results in Table 5 that Medium Formula 2 contains no CaCl₂, NaCl, NH₄Cl, or resazurin. It also excludes acidic trace element solution (containing FeCl₂, H₃BO₃, ZnCl₂, CuCl₂ · 2H₂O, MnCl₂, CoCl₂, and NiCl₂) and alkaline trace element solution (containing Na₂SeO₃, Na₂WO₄·2H₂O, and Na₂MoO₄·2H₂O). Meanwhile, the vitamin solution (containing thiamine, riboflavin, nicotinic acid, calcium pantothenate, pyridoxine, biotin, and p-aminobenzoic acid) was replaced with cobalamin. Under such conditions, neither the OD₆₀₀ value nor the viable cell count decreased markedly. By contrast, both the OD₆₀₀ value and viable cell count of the optimized Medium Formula 3 and Formula 4 increased significantly (p < 0.01) compared with the control group. Table 5: Effects of Optimized Media and Original Medium on the Growth of *Akkermansia muciniphila*

| **Formula 1 (control)** | | **Formula 2** | | **Formula 3** | | **Formula 4** | | **Formula 5** | |
|---|---|---|---|---|---|---|---|---|---|
| **Component** | **g/L** | **Component** | **g/L** | **Component** | **g/L** | **Component** | **g/L** | **Component** | **g/L** |
| Glucose | 4.5 | Glucose | 4.5 | Maltose | 8 | 2'-Fucosylla ctose | 11 | Dextran | 4 |
| N-Acetylglucosamine | 5.5 | N-Acetylglucosamine | 5.5 | N-Acetylglucosamine | 12 | N-Acetylmannosamine | 10 | N-Acetylmannosamine | 15 |
| Soy peptone | 16 | Soy peptone | 16 | Yeast extract | 11 | Yeast extract | 12 | Yeast extract | 20 |
| Threonine | 4 | Threonine | 4 | Threonine | 6 | Threonine | 4 | Threonine | 8 |
| / | / | | | Phenylalanine | 1.2 | Phenylalanine | 0.5 | Phenylalanine | 0.5 |
| / | / | | | Tryptophan | 0.5 | Chitin | 2 | Tryptophan | 0.5 |
| CaCl₂ | 0.11 | / | | / | | | | Chitosan | 5 |
| MgCl₂ | 0.1 | MgCl₂ | 0.1 | MgCl₂ | 0.12 | MgCl₂ | 0.12 | MgCl₂ | 0.1 2 |
| NaCl | 0.3 | / | / | / | / | / | / | | |
| NH₄Cl | 0.3 | / | / | / | / | / | / | | |
| Na₂S·9H₂O | 0.25 | Na₂S·9H₂O | 0.25 | CH₄N₂O₂S | 0.11 | Na₂S₂O₄·5H₂O | 0.25 | Na₂S₂O₄·5H₂O | 0.25 |
| Resazurin | 0.0005 | / | / | / | / | / | / | / | / |
| KH₂PO₄ | 0.4 | KH₂PO₄ | 0.4 | KH₂PO₄ | 2 | / | / | KH₂PO₄ | 2 |
| Na₂HPO₄ | 0.53 | Na₂HPO₄ | 0.53 | K₂HPO₄ | 2 | Na₂HPO₄ | 2 | / | / |
| NaHCO₃ | 4 | NaHCO₃ | 4 | Na₂CO₃ | 2.5 | NaHCO₃ | 4 | NaHCO₃ | 5 |
| Acidic trace element solution | 1 mL | / | / | / | / | / | / | / | / |
| Alkaline trace element solution | 1 mL | / | / | / | / | / | / | / | / |
| Vitamin solution | 1 mL | Cobalamin | 0.0001 | Cobalamin | 0.0002 | Cobalamin | 0.0002 | Cobalamin | 0.0002 |
| OD₆₀₀ | 0.7719 | | 0.7688 | | **1.198**** | | **1.102**** | | **1.1579 **** |
| Viable cell count (cfu/mL) | 1.74× 10⁹ | | 1.68 ×10⁹ | | **4.2 ×10^{9**}** | | **3.5 ×10^{9**}** | | **4.1 ×10^{9**}** |

| | | | | | | | | | |
|---|---|---|---|---|---|---|---|---|---|
| Note: * indicates p < 0.05 versus the control group; ** indicates p < 0.01 versus the control group. | | | | | | | | | |

### Example 7: Effects of pH on High-Density Fermentation of Akkermansia muciniphila

Formula 3 in Table 5 of Example 6 (hereinafter referred to as Medium No. 3) was used as the culture medium for high-density fermentation.

The specific experimental procedures were as follows: The strain preserved at -80 °C was removed and inoculated at an inoculum size of 1% into test tubes containing 5 mL of the control medium and pre-cultured for 24 h. Subsequently, the bacterial suspension was transferred at an inoculum size of 1% into 100 mL of Medium No. 3 and subcultured for another 24 h. Afterwards, the bacterial suspension was transferred into a fermenter filled with Medium No. 3 at an inoculum size of 1%. The culture conditions were set as follows: temperature 37 °C, stirring speed 150 rpm, and continuous slow aeration with a mixed gas of 10% H₂, 10% CO₂, and 80% N₂. The pH was controlled at 7.5, 7.0, 6.5, or 6.0, respectively, with one group without pH control.

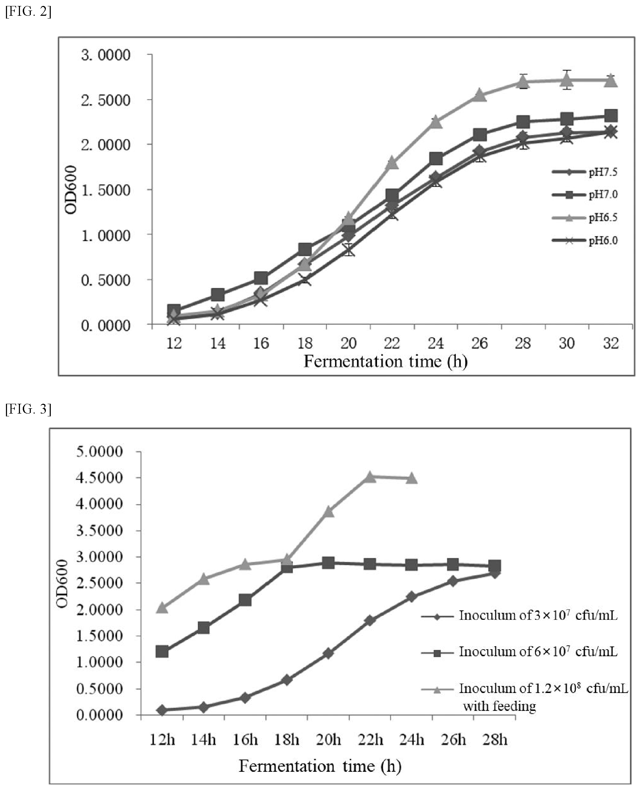

The initial pH of the fermentation medium was approximately 8.5. 3 M hydrochloric acid was used to adjust the pH to the preset values, and fermentation was carried out overnight after inoculation. Starting from the 12th hour of fermentation, samples were collected every 2 h. After four-fold dilution, the OD₆₀₀ value was measured using a microplate reader. The results are presented in FIG. 2. As shown in FIG. 2, the biomass of *Akkermansia muciniphila* increased as pH decreased, except for the group at pH 6.0. The optimal growth was observed at pH 6.5. Under this condition, the culture reached the stationary phase at 28 h with an OD₆₀₀ of 2.7007. When the fermentation was prolonged to 40 h, the OD₆₀₀ was 2.6992 (data not shown). For the group at pH 7.0, the growth rate slowed down at 26 h, and the culture entered the stationary phase at 30 h. The growth curves of the groups at pH 6.0 and pH 7.5 were highly consistent, yet their growth performance was inferior to that of the pH 7.0 group. In the group without pH control, the pH decreased to around 5.35 after 40 h of culture, and the corresponding OD₆₀₀ was 1.5183 (growth curve not shown).

### Example 8: Effects of Inoculum Size and Fed-Batch Fermentation on High-Density Fermentation of Akkermansia muciniphila

In this example, Medium No. 3 was used as the high-density culture medium.

The specific experimental procedures were as follows: The strain preserved at -80 °C was inoculated into test tubes containing 5 mL of the control medium at an inoculum size of 3×10⁷ cfu/mL and pre-cultured for 36 h. The bacterial suspension was then transferred at an inoculum size of 1% into 100 mL of Medium No. 10 and subcultured for another 36 h. Subsequently, the bacterial suspension was transferred into fermenters containing Medium No. 3 at three different inoculum sizes: 3×10⁷ cfu/mL, 6×10⁷ cfu/mL and 1.2×10⁸ cfu/mL. Feeding was initiated once the cultures entered the stationary phase. The feed medium consisted of 300 mL carbon sources (maltose : N-acetylglucosamine = 4 : 6) with a final concentration of 240 g/L. The culture conditions were set as follows: temperature 37 °C, stirring speed 150 rpm, continuous slow aeration with a mixed gas of 10% H₂, 10% CO₂, and 80% N₂, and pH maintained at 6.5.

The results are shown in FIG. 3. It was observed that a larger inoculum size led to faster cell growth and earlier entry into the stationary phase. The culture with an inoculum size of 3×10⁷ cfu/mL had not reached the stationary phase after 28 h of fermentation. The group with an inoculum size of 6×10⁷ cfu/mL entered the stationary phase at 20 h. For the group with an inoculum size of 1.2×10⁸ cfu/mL, the stationary phase was reached at 16 h, and feeding started at the 18th hour. Afterwards, the OD₆₀₀ value increased rapidly and peaked at 4.52 at 22 h of fermentation, with no further increase thereafter. At this time, the maximum viable cell count reached 2×10¹⁰ cfu/mL.

### Example 9: Comparison of Precipitate Formation in Media after Sterilization

After sterilization and cooling to room temperature, the control medium described in Example 6 had a pH of approximately 8.2. A large amount of precipitate formed in this medium due to its high Ca²⁺ content. As demonstrated in Example 7, the optimal pH for the growth of *Akkermansia muciniphila* is 6.5. When the pH of the control medium was adjusted to 6.5, partial precipitate still remained. The medium turned completely clear and transparent when the pH was further lowered to 5.5. In contrast, Medium No. 3 showed an OD₆₀₀ value of only 0.0068 after sterilization, and no precipitate was generated. The blank OD₆₀₀ values of the control medium and Medium No. 3 at different pH levels are presented in Table 6. The results indicate that Medium No. 3 can effectively eliminate precipitate formation during high-temperature and high-pressure sterilization.

**Table 6: Changes in OD₆₀₀ of Sterilized Control Medium and Medium No. 3 at Different pH Values**

| | pH8.2 | pH6.5 | pH6.0 | pH5.5 |
|---|---|---|---|---|
| Control Medium | 0.3579 | 0.1155 | 0.0647 | 0.0088 |
| Medium No. 3 | 0.0361 | 0.0068 | \ | \ |

### Example 10: Effects of Culture in Different Media on Downstream Processing Technology

The control medium and optimized medium (Medium No. 3) were respectively prepared according to the medium formulas listed in Table 5 of Example 6. The strain was inoculated at an inoculum size of 1% into test tubes containing 5 mL of the control medium and cultured for 36 h. Afterwards, the bacterial suspension was transferred at an inoculum ratio of 1% into 100 mL of the control medium and Medium No. 10, respectively. The culture was carried out at 37 °C under an atmosphere of 10% H₂, 10% CO₂, and 80% N₂. After 36 h of culture, the bacterial suspensions were transferred into 50 mL centrifuge tubes and centrifuged using a centrifuge (H1750R, Xiangyi Instrument, China) at 10,000 rpm for 15 min.

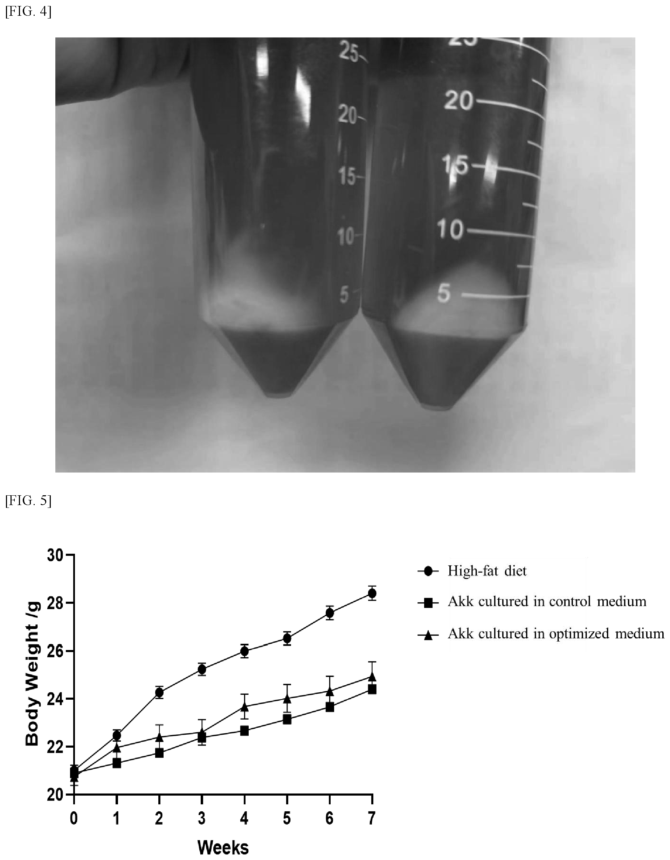

As shown in Example 7, the pH of the fermentation broth dropped to 5.35 after fermentation without pH control, which was lower than 5.5. At this pH, all precipitates in the fermentation broth were fully dissolved, and no calcium salt precipitates existed in the bacterial pellets obtained by centrifugation. The OD₆₀₀ value of the supernatant harvested from the control medium culture was 0.2433, indicating that a large number of bacterial cells failed to be recovered via centrifugation. In contrast, the OD₆₀₀ of the supernatant from Medium No. 3 culture was merely 0.0095 (less than 0.01), which meant almost no cell loss occurred. The centrifugation results are presented in FIG. 4. The centrifuge tube on the left shows the result from the control medium, with rough and scattered pellet edges, reflecting poor centrifugal precipitation performance. The tube on the right displays the result from Medium No. 3, featuring smooth and rounded pellet edges, which demonstrates superior centrifugal precipitation efficiency.

### Example 11: Effects of Bacterial Cells Cultured in Different Media on Body Weight in Mice

The control medium and the optimized medium (Medium No. 3) were prepared, respectively, according to the medium formulas listed in Table 5 of Example 6. The strain preserved at -80 °C was removed and inoculated at an inoculum size of 1% into test tubes containing 5 mL of the control medium and pre-cultured for 36 h. Subsequently, the bacterial suspension was transferred at an inoculum size of 1% into 100 mL of the control medium and Medium No. 3, respectively, and cultured under an atmosphere of 10% H₂, 10% CO₂, and 80% N₂ for 36 h.

The fermentation broths obtained from the control medium and Medium No. 3 were respectively centrifuged, and the resulting cell pellets were washed three times with PBS and then stored in 25% glycerol at -80 °C for later use. A hyperlipidemia mouse model was established, with three groups of mice (10 mice per group), which were fed a high-fat diet. Among them, two groups were fed with a high-fat diet and simultaneously administered intragastrically with *Akkermansia muciniphila* cultured in the control medium and Medium No. 3, respectively, at a dose of 2×10⁸ cfu/mouse/day. The remaining group served as the high-fat control group and was administered with an equal volume of PBS. Body weight was measured weekly. The effects of bacterial cells cultured in the two media on body weight in mice were compared, and the results are shown in FIG. 5 (where "Akk" in the figure is the abbreviation for *Akkermansia muciniphila*).

It can be seen from FIG. 5 that compared with the high-fat control group, *Akkermansia muciniphila* cultured in both the control medium and Medium No. 3 were able to reduce body weight in mice, with statistically significant differences (p < 0.05). No significant difference in body weight was observed between the two groups of mice administered bacterial cells cultured in the control medium and Medium No. 3 (p > 0.05). This indicates that *Akkermansia muciniphila* cultured in Medium No. 3 exhibited no alteration in its physiological functions compared with that cultured in the control medium.

### Example 12: Genomic Comparison of Akkermansia muciniphila

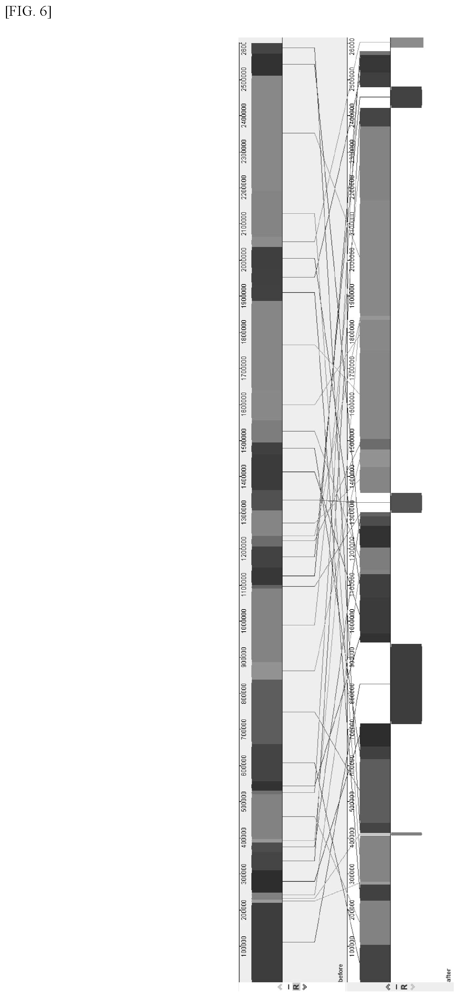

*Akkermansia muciniphila* was cultured in the control medium and Medium No. 3 according to the method described in Example 6. The resulting fermentation broths were centrifuged to collect the cell pellets, which were then subjected to whole-genome sequencing for collinearity analysis (collinearity refers to the distribution or arrangement of homologous genes within or between species). The results are shown in FIG. 6. In FIG. 6, the upper panel represents the results obtained from *Akkermansia muciniphila* cultured in the control medium, and the lower panel represents the results obtained from the strain cultured in Medium No. 3. As can be seen from FIG. 6, the genome annotation results of the strains from the two sources were completely identical. The fastani value for genomic comparison between the strains from the two sources was 99.9999%, indicating that the average nucleotide identity between the genomes of the strains from the two sources was almost completely consistent, thereby demonstrating that the optimized medium does not induce genomic changes in *Akkermansia muciniphila*.

## Claims

1. A culture medium for culturing *Akkermansia muciniphila*, comprising:
a carbohydrate at a concentration of 2-200 g/L, preferably 2-50 g/L, more preferably 4-20 g/L, wherein the carbohydrate is selected from disaccharide, oligosaccharide, polysaccharide, and any combination thereof;
a glucosamine and/or glucosamine polymer at a concentration of 2-200 g/L, preferably 2-50 g/L, more preferably 5-20 g/L, wherein the glucosamine and/or glucosamine polymer is selected from D-glucosamine, D-glucosamine hydrochloride, N-acetyl-D-glucosamine, N-acetyl-D-galactosamine, N-acetyl-D-mannosamine, chitosan oligosaccharide, chitosan, chitin, and any combination thereof;
a non-animal-derived nitrogen source at a concentration of 5-200 g/L, preferably 5-50 g/L, more preferably 10-20 g/L, wherein the non-animal-derived nitrogen source is selected from soybean peptone, wheat peptone, yeast extract, yeast peptone, and any combination thereof;
L-threonine at a concentration of 0.1-20 g/L, preferably 2-10 g/L, more preferably 4-8 g/L;
a reducing agent at a concentration of 0-50 mM, preferably 0.1-10 mM, more preferably 1-10 mM, wherein the reducing agent is selected from L-cysteine, L-cysteine hydrochloride, SO₃²⁻, S₂O₃²⁻, S₂O₄²⁻, thiourea dioxide (CH₄N₂O₂S), and any combination thereof;
cobalamin at a concentration of 0-1 g/L, preferably 0.1-10 mg/L;
Mg²⁺ at a concentration of 0-10 mM, preferably 0-5 mM; and
a buffer salt at a concentration of 0-10 g/L, preferably 2-7 g/L, wherein the buffer salt is selected from dipotassium hydrogen phosphate, potassium dihydrogen phosphate, sodium dihydrogen phosphate, sodium bicarbonate, sodium carbonate, and any combination thereof.

2. The culture medium according to claim 1, wherein
the disaccharide is selected from lactose, maltose, isomaltulose, cellobiose, turanose, melibiose, and any combination thereof;
the oligosaccharide is selected from galacto-oligosaccharide, fructo-oligosaccharide, mannan-oligosaccharide, isomalto-oligosaccharide, malto-oligosaccharide, soybean oligosaccharide, human milk oligosaccharide (e.g., 2'-fucosyllactose, 3'-sialyllactose, lacto-N-neotetraose, lacto-N-triose), and any combination thereof;
the polysaccharide is selected from dextrin (maltodextrin, resistant dextrin, cyclodextrin), starch, modified starch (e.g., pregelatinized starch, soluble starch, etc.), dextran, and any combination thereof;
the glucosamine and/or glucosamine polymer is N-acetyl-D-glucosamine, N-acetyl-D-mannosamine, chitosan, chitosan oligosaccharide, chitin, or any combination thereof;
the non-animal-derived nitrogen source is yeast extract;
the reducing agent is S₂O₃²⁻, or a sodium salt, potassium salt, or hydrate thereof, S₂O₄²⁻, or a sodium salt, potassium salt, or hydrate thereof, thiourea dioxide (CH₄N₂O₂S), or any combination thereof; preferably, the reducing agent is S₂O₃²⁻, or the sodium salt, potassium salt, or hydrate thereof, such as Na₂S₂O₃·5H₂O.

3. The culture medium according to claim 1 or 2, wherein the culture medium further comprises 0-2 g/L, preferably 0.5-2 g/L, of other amino acids selected from L-phenylalanine, L-tryptophan, or any combination thereof.

4. The culture medium according to claim 1 or 2, wherein a concentration of potassium dihydrogen phosphate, dipotassium hydrogen phosphate, or sodium dihydrogen phosphate each independently ranges from 0 to 4.5 g/L, preferably from 0 to 2 g/L; and a concentration of sodium bicarbonate ranges from 1 to 10 g/L, preferably from 1 to 5 g/L.

5. The culture medium according to claim 4, wherein the culture medium comprises 4-15 g/L carbohydrate, 10-20 g/L glucosamine and/or glucosamine polymer, 10-20 g/L yeast extract, 0.5-1.2 g/L phenylalanine, 0.25 g/L sodium thiosulfate pentahydrate or an equivalent amount of sodium thiosulfate or 0.11 g/L CH₄N₂O₂S, 4-8 g/L L-threonine, 0.1 mg/L cobalamin; and optionally, 0.5 g/L tryptophan, 0.1-0.12 g/L magnesium chloride, and 6-7 g/L buffer salt.

6. A method for culturing *Akkermansia muciniphila*, comprising culturing *Akkermansia muciniphila* in the culture medium according to any one of claims 1-5; preferably, the culture is static culture, high-density culture, or static culture followed by high-density culture.

7. The method according to claim 6, wherein the static culture comprises culturing at 36 °C-38 °C for 12-36 h with 2-3 passages; preferably, the 2nd and 3rd passages are carried out using the culture medium according to any one of claims 1-6.

8. The method according to claim 6 or 7, wherein the high-density culture comprises inoculating *Akkermansia muciniphila* into the culture medium at an inoculum size of 3×10⁷ cfu/mL to 3×10⁸ cfu/mL, preferably 3×10⁷ cfu/mL to 1.5×10⁸ cfu/mL, and culturing at a temperature of, for example, 20 °C-40 °C, preferably 36 °C-38 °C, for a period of time, for example, 24-36 h; more preferably, the high-density culture further comprises controlling a pH of the culture medium during a culture process to 6-8, preferably to 6.0-7.5, and most preferably to 6.5.

9. The method according to claim 6 or 7, wherein the high-density culture is carried out in a fed-batch fermentation mode; the feed is, for example, a complete feeding medium or a semi-fed-batch medium, such as an incomplete medium comprising a carbon source (e.g., carbohydrate, glucosamine and/or glucosamine polymer) and a nitrogen source; for example, continuous feeding is performed at a feeding rate of 1 mL·L⁻¹·h⁻¹ to 6 mL·L⁻¹·h⁻¹, preferably 1.2 mL·L⁻¹·h⁻¹.

10. A strain of *Akkermansia muciniphila* obtained by the method according to any one of claims 6-9, wherein the strain was deposited on September 13, 2023, at the China General Microbiological Culture Collection Center (CGMCC), with the deposit address being No. 3, Yard 1, Beichen West Road, Chaoyang District, Beijing, China, and with the deposit accession number CGMCC NO. 40786.
